# EUROPÄISCHE PATENTSCHRIFT

(11) **EP 2 135 639 B1**
(45) Veröffentlichungstag und Bekanntmachung des Hinweises auf die Patenterteilung: **22.06.2011**
(21) Anmeldenummer: 09159865.6
(22) Anmeldetag: 11.05.2009
(51) Int. Cl.: A61N 1/08, A61N 1/37, A61N 1/39, H01R 13/66, H02H 9/04

(54) **Überspannungsschutzelement**
Electrical surge protection element
Elément de protection contre les surtensions

(30) Priorität: 10.06.2008 DE 102008002330
(43) Veröffentlichungstag der Anmeldung: 23.12.2009
(73) Patentinhaber: Biotronik CRM Patent AG, 6341 Baar (CH)
(72) Erfinder: Gromotka, Bernhard, 12359, Berlin (DE); Mader, Dirk, 13465, Berlin (DE)
(74) Vertreter: Lindner-Vogt, Karin L.

(56) Entgegenhaltungen:
- EP-A- 0 426 969
- WO-A-2006/057587
- DE-A1-102006 033 925
- US-A- 4 440 172
- US-A- 4 595 009
- US-A1- 2007 049 993

## Beschreibung

Die Erfindung betrifft ein Überspannungsschutzelement als Bestandteil eines zum Einsatz an oder in einem menschlichen oder tierischen Körper vorgesehenen medizinischen Gerätes gemäß dem Oberbegriff von Patentanspruch 1.

In den nachfolgenden Ausführungen wird weitgehend davon ausgegangen, dass das medizinische Gerät zum Einsatz an oder in einem menschlichen oder tierischen Körper vorgesehen ist und folglich wenigstens eine Schnittstelle aufweist, über welche das Gerät entweder Signale an eine vorbestimmte Körperstelle abgibt oder von dieser aufnimmt. Hierbei muss die bezeichnete Schnittstelle nicht eine Körper-Gerät-Schnittstelle sein, sondern kann beispielsweise auch einen Anschluss des medizinischen Geräts betreffen, welcher mit weiteren Mess- oder Wirkvorrichtungen verbunden werden kann.

Medizinischen Geräten, insbesondere Elektrostimulatoren und darunter spezielle Herzschrittmachern, wird aufgrund ihrer lebensrettenden und lebenserhaltenden Eigenschaften heutzutage eine immer größere Bedeutung beigemessen. Manche medizinische Geräte können komplexe Körperfunktionen von Patienten übernehmen, oder sie unterstützen diese durch entsprechend konditionierte elektrische Signale, mit welchen vorbestimmte Körperpartien beschickt und energetisiert werden. Weiterhin werden medizinische Geräte vielfach dafür benutzt, elektrische Signale am Körper eines Patienten aufzunehmen und nach entsprechender Verarbeitung zur Diagnose oder für Therapieverfahren einzusetzen. Vielfach haben moderne medizinische Geräte therapeutische und diagnostische Funktionen, wie dies etwa bei modernen Herzschrittmachern der Fall ist, welche die Herzmuskeltätigkeit einerseits mittels speziell dafür angepasster Messvorrichtungen überwachen und diese andererseits auch steuern können.

Gemein ist derartigen medizinischen Geräten, dass sie eine Körper-Gerät-Schnittstelle aufweisen, über welche sie mit einem menschlichen oder auch tierischen Körper zur elektrischen Signalübermittlung in Kontakt stehen. Vergleichbare Körper-Gerät-Schnittstellen liegen etwa auch bei externen wie implantierbaren Defibrillatoren, Stimulationsanordnungen zur Stimulation des Hörnervs oder sonstigen implantierbaren Mess- und Übertragungsanordnungen zur intrakorporalen Erfassung und Auswertung und/oder externen Übertragung von Messwerten physiologischer Größen vor.

Kommt es zur gleichzeitigen Anwendung mehrerer derartiger medizinischer Geräte bei einem Patienten, besteht die Gefahr der gegenseitigen elektrischen Beeinflussung der Funktionsfähigkeit der Geräte, welche möglicherweise eine Störung oder Beschädigung darin enthaltener Bauteile durch elektrische Überspannungen hervorrufen kann. Dies kann etwa vorkommen, wenn eine elektrische Spannung eines ersten medizinischen Gerätes über den Körper des Patienten in eine Körper-Gerät-Schnittstelle eines zweiten medizinischen Gerätes eingekoppelt wird. Resultieren die eingekoppelten Spannungssignale aus an den Körper angelegten relativ hohen Spannungen, wie sie beispielsweise von Defibrillatoren oder auch HF-chirurgischen Geräten ausgegeben werden können, kann es zur Zerstörung von internen elektronischen Bauteilen eines zweiten medizinischen Gerätes kommen, falls die hochspannungsbeaufschlagten Bauteile nicht eine geeignete Spannungsfestigkeit aufweisen.

Um interne Überspannungen durch Fremdeinkopplung zu vermeiden, verwenden viele elektrotechnische Geräte Überspannungsschutzschaltungen, welche es erlauben, in eine Schnittstelle eingekoppelte Überspannungen auf einen vorbestimmten Minimalwert zu vermindern und somit elektronische Komponenten vor zu hohen Spannungsdifferenzen und daraus folgenden Zerstörungen durch elektrischen Spannungsdurchschlag zu schützen. Derartige Überspannungsschutzschaltungen haben sich in der elektronischen Schaltungstechnik seit Jahrzehnten in vielerlei Anwendungen bereits bewährt.

Eine herkömmliche Überspannungsschutzschaltung umfasst etwa in einer Eingangsstufe eines Gerätes eine Reihenschaltung aus einem Widerstand und einer Zener-Diode, in welcher die eingekoppelte Überspannungsenergie in Wärmeenergie umgesetzt wird.

Eine weiteres Beispiel einer Überspannungsschutzschaltung, welche sowohl ein Spannungsbegrenzungselement umfasst, das zwischen zwei Leitungseingänge einer zu schützenden elektronischen Schaltung vorgesehen ist, als auch eine Strombegrenzungsvorrichtung, die mit dem Spannungsbegrenzungselement in Reihe geschaltet ist, ist in der US 5,751,531 A offenbart. Die Strombegrenzungsvorrichtung kann als MOS-Transistor ausgeführt sein, dessen Gate- und Source-Anschlüsse miteinander kurzgeschlossen sind und der lediglich mit über seinen Source- und seinen Drain-Anschluss verschaltet ist. Das Spannungsbegrenzungselement ist typischerweise als Zener-Diode ausgeführt.

Eine ähnliche Überspannungsschutzschaltung, welche zwei zueinander in Umkehrrichtung geschaltete 60-Volt-Zener-Dioden umfasst, die zwischen einem Eingangs- und einem Erdungsanschluss geschaltet sind, ist in der US 4,661,979 offenbart. Weiterhin umfasst diese Überspannungsschutzschaltung zwei zueinander in Umkehrrichtung geschaltete Dioden innerhalb eines integrierten Schaltkreises, welche zwischen den zwei Eingängen des integrierten Schaltkreises und einem internen Masseanschluss vorgesehen sind, und eine in Vorwärtsrichtung geschaltete Diode, welche zwischen dem internen Masseanschluss und einer Spannungsversorgung geschaltet ist. Weitere ähnliche Überspannungsschutzschaltungen, die zusätzlich noch eine kapazitive Ankoppelung der Überspannungsschutzelemente aufweisen, sind in WO 2006/057587 A1, US 2007/0049993 A1 und EP 0 426 969 A2 offenbart.

Oben beschriebene bekannte Überspannungsschutzschaltungen ermöglichen bei Anliegen eines Überspannungssignals die Ausbildung einer Kurzschlussschaltung, über welche die für weitere elektronische Bauteile möglicherweise zur Schädigung führenden hohen Spannungen und Energien abgeleitet werden.

Im Falle der Verwendung eines Defibrillators an einem mit weiteren Körper-Gerät-Schnittstellen eines medizinischen Geräts versehenen Körpers würde die Ausbildung einer Kurzschlussschaltung in den zu schützenden medizinischen Geräten jedoch dazu führen, dass die Defibrillationsenergie nicht voll am Herzen des Patienten wirksam wird, sondern über den Kurzschluss von dem Körper des Patienten abgeführt würde und damit für die Defibrillation verloren ginge. Dies ist insbesondere der Fall, falls die Körper-Gerät-Schnittstelle in der Nähe der Körperregion angeordnet ist, welche mit der Defibrillationsenergie beaufschlagt wird, wie dies bei Stimulatoren und Herzschrittmachern der Fall ist.

Es ist daher wünschenswert, dass die bei einer Defibrillation über die Defibrillatorelektroden ausgegebene Energie weitgehend vollständig auf den Körper des Patienten einwirkt und nicht über eine Körper-Gerät-Schnittstelle eines weiteren medizinischen Geräts ungenutzt abfließt.

Der vorliegenden Erfindung liegt also die Aufgabe zugrunde, ein Überspannungsschutzelement als Bestandteil eines zum Einsatz an oder in einem menschlichen oder tierischen Körper vorgesehen medizinischen Gerätes vorzuschlagen, welches den beschriebenen Nachteil der aus dem Stande der Technik bekannten Überspannungsschutzschaltungen vermeidet.

Diese Aufgabe wird durch ein Überspannungsschutzelement mit den Merkmalen des vorliegenden Patentanspruches 1 gelöst. Zweckmäßige Fortbildungen des Erfindungsgedankens sind Gegenstand der abhängigen Ansprüche.

Die Erfindung basiert auf dem Gedanken, Umformungsmittel in einem Überspannungsschutzelement vorzusehen, welche dazu ausgebildet sind, externe Überspannungssignale an einer Schnittstelle eines medizinischen Geräts in wenigstens einen internen Spannungspuls zu überführen. Hierdurch wird einerseits ein direkter Spannungskurzschluss über die Dauer des externen Überspannungssignals vermieden, andererseits kann durch eine geeignete Wahl der Umformungsmittel der in das medizinische Gerät eingekoppelte über die zeitliche Dauer des externen Überspannungssignals integrierte Gesamtenergiebetrag derart herabgesetzt werden, dass dieser weitere elektronische, mit dem Überspannungsschutzelement verschaltete Komponenten des medizinischen Geräts nicht beschädigt.

Die Schnittstelle des medizinischen Geräts kann hierbei mit der bereits bezeichneten Körper-Gerät-Schnittstelle identisch oder aber auch als eine weitere Schnittstelle eines medizinischen Geräts ausgeführt sein. Insbesondere kann diese Schnittstelle ein Patientenanschluss eines medizinischen Geräts sein, welcher zum Anschluss von Elektroden eines Stimulators oder eines Herzschrittmachers vorgesehen ist.

Das erfindungsgemäße Überspannungsschutzelement sieht weiterhin Begrenzungsmittel vor, welche dazu vorgesehen sind, einen Spannungsabfall an wenigstens einem elektronischen Bauteil des medizinischen Geräts auf einen vorbestimmten Grenzwert zu begrenzen. Hierbei kann die verbleibende Spannungsbelastung an die Spannungsfestigkeit der elektronischen Bauteile in dem medizinischen Gerät angepasst werden. Eine solche Anpassung verhindert eine Beschädigung oder Beeinträchtigung der elektronischen Bauteile. Diese Spannungsbegrenzung erschließt Möglichkeiten zu den angestrebten Verbesserungen gegenüber herkömmlichen, rein auf die Herbeiführung einer elektrischen Kurzschlussschaltung bei Auftreten von Überspannungen beruhenden Schutzschaltungen.

Eine erste Ausführung der Erfindung sieht vor, dass die Umformungsmittel den Begrenzungsmitteln in Bezug auf die Ausbreitungsrichtung des externen Überspannungssignals an der Schnittstelle in Reihe vorgeschaltet sind.

In einer weiteren, zweckmäßigen und kostengünstigen Ausführungsform umfassen die Umformungsmittel wenigstens einen hochspannungsfesten Kondensator. Wird ein Kondensator mit einem externen Überspannungssignal mit zeitlicher Anstiegs- und/oder Abklingcharakteristik beaufschlagt, wird der Kondensator in einen Ladezustand und darauf folgenden Entladezustand versetzt, wobei Gleichspannungsanteile des externen Spannungssignals ausgefiltert werden und nur ein interner Puls in das Überspannungsschutzelement eingekoppelt werden kann. Ist das externe Überspannungssignal ein zeitlich kurzer Defibrillationspuls, führt dieser ebenfalls zu einem kurzzeitigen Auf und Entladen des Kondensators.

Durch geeignete Dimensionierung des Kondensators kann einerseits die Zeitcharakteristik des eingekoppelten Spannungspulses und andererseits der Pegel der eingekoppelten Spannung vorbestimmt werden. Folglich erlaubt es das Vorsehen eines entsprechend dimensionierten Kondensators, sowohl die zeitliche als auch gleichzeitig die energetische Umwandlung des externen Spannungssignals zu beeinflussen. Dabei muss jedoch gewährleistet sein, dass der Kondensator nicht die Signalsauskopplung oder Signaleinkopplung der Nutzsignale des medizinischen Geräts beeinträchtigt. Durch eine entsprechende Dimensionierung des Kondensators kann die in das medizinische Gerät eingekoppelte Energiemenge und Energiedichte beeinflusst werden, andererseits können zeitlich veränderliche Nutzsignale, wie etwa Stimulationspulse eines Herzschrittmachers, weitgehend widerstandslos über die Schnittstelle ausgekoppelt werden. Ein Kondensator stellt folglich einen effizienten Überspannungsschutz dar, ohne gleichzeitig die Funktionsweise des medizinischen Geräts zu beeinträchtigen.

Es ist vorgesehen, dass die Begrenzungsmittel oberhalb eines vorbestimmten Betrags eines Spannungswertes im Wesentlichen niederohmig stromleitend werden und einen eingangseitigen elektrischen Verbindungspfad für einen Ausgleich der an weiteren elektronischen Bauteilen des medizinischen Geräts anliegenden Spannungen ausbilden.

Die Definition der anliegenden Spannungen ist hierbei im Sinn einer Spannungsdifferenz zu verstehen, welche durch den Schaltungsaufbau bzw. die Verschaltung der weiteren Bauteile des medizinischen Geräts festgelegt ist. So wird etwa durch Veränderung eines Potentialwertes auch bei gleich bleibendem absoluten Spannungsbetrag des internen Spannungspulses die Höhe der relativen Spannungsamplitude, d. h. die Höhe der Spannungsdifferenz, variiert, welche an weiteren Bauteilen des medizinischen Geräts anliegen kann. Durch Ausbildung eines eingangsseitigen elektrischen Verbindungspfads kann der Spannungsabfall an in dem medizinischen Gerät vorgesehenen elektronischen Bauteilen derart beeinflusst werden, dass keine schädigenden Potentialdifferenzen auftreten, welche möglicherweise einen Spannungsdurchbruch in den Bauteilen bewirken könnten. Hierbei kann der elektrische Verbindungspfad einen Ausgleich von an den Bauteilen des medizinischen Geräts anliegenden Potentialen erlauben und bewirkt folglich die Vermeidung von schädigenden Potentialdifferenzen.

Die Begrenzungsmittel werden im Wesentlichen niederohmig stromleitend, wenn eine Spannung anliegt, deren Betrag einen vorbestimmten Betrag einer Nutzspannung, insbesondere einer maximalen Ausgabespannung oder Messspannung des medizinischen Geräts, übersteigt und bilden einen eingangsseitigen elektrischen Strompfad für einen Ausgleich der an weiteren elektronischen Bauteilen des medizinischen Geräts anliegenden Spannungen aus. Folglich kann gewährleistet werden, dass keinerlei Kurzschlussschaltung durch die Begrenzungsmittel erfolgt, solange das medizinische Gerät regulär benutzt wird.

Die Begrenzungsmittel können gemäß der vorliegenden Ausführungsform entsprechend der Nutzspannung des medizinischen Gerätes so ausgewählt werden, dass alle Nutzspannungen unterhalb einer Schwelle liegen, bei der die Begrenzungsmittel im Wesentlichen stromleitend werden. Für alle höheren Spannungen, insbesondere eingekoppelte externe Überspannungssignale, werden die Begrenzungsmittel im Wesentlichen stromleitend, was eine Schädigung von elektronischen Bauteilen des medizinischen Geräts durch Potentialausgleich verhindert. Die Spannungsschwelle kann in anderen Ausführungsformen der Begrenzungsmittel auch um einen vorbestimmten Betrag über den im regulären Betrieb höchsten auftretenden Nutzspannungsbetrag liegen, um zusätzliche Sicherheit bei Auftreten von unerwarteten Spannungsschwankungen zu gewährleisten. Ein typischer derartiger Schwellenwert eines das ausführungsgemäße Überspannungsschutzelement umfassenden Herzschrittmachers kann z. B. im Bereich von 10 V bis 20 V liegen.

Die Begrenzungsmittel umfassen wenigstens eine Spannungsbegrenzungsschaltung, welche in Bezug auf ihre Anordnung symmetrisch ist. Aufgrund der Symmetrie wird eine leichtere Dimensionierung der Bauteile der Spannungsbegrenzungsschaltung ermöglicht, da in deren Anordnung typischerweise nur ein Teil der Schaltung dimensioniert zu werden braucht. Fernerhin bedarf es bei der Schnittstelle des medizinischen Gerätes mitunter keiner Berücksichtigung der Polarität der Anschlüsse, da in Bezug auf in das Überspannungsschutzelement eingekoppelte Spannungspulse keine elektrischen Vorzugsorientierungen vorliegen. Mithin können die Begrenzungsmittel auch unabhängig von der Spannungspolarität sein, und die Funktionstüchtigkeit der Begrenzungsmittel kann sowohl für positive wie auch negative Überspannungssignale gewährleistet werden.

Die Begrenzungsmittel umfassen wenigstens zwei parallel zur Schnittstelle und zueinander in Reihe geschaltete Zener-Dioden; wobei eine Zener-Diode in Sperrrichtung und eine in Durchlassrichtung geschaltet ist. Zener-Diode verhalten sich typischerweise in Durchlassrichtung wie normale Dioden, in Sperrrichtung werden sie jedoch ab einer vorbestimmten Spannung, der Durchbruchspannung, niederohmig leitend. Dementsprechend eignen sich Zener-Dioden zur Darstellung von Spannungsbegrenzungsschaltungen, welche ab einem vorgegebenen Spannungsgrenzwert ihr Leitungsverhalten ändern und so Stromfluss und Spannungsabfall maßgeblich in einer Schaltung zur Spannungsbegrenzung beeinflussen können.

Durch das Vorsehen zweier zueinander in Reihe geschalteter Zener-Dioden, von denen eine in Durchlass- und die andere in Sperrrichtung geschaltet ist, wird es unerheblich, welche Spannungspolarität ein eingekoppelter interner Spannungspuls aufweist. Während eine der beiden Zener-Dioden negative, interne Spannungspulse unterhalb eines Durchbruchspannungswertes sperren kann, jedoch positiven internen Spannungspulsen eines vorbestimmten Betrages nur einen geringen Leitungswiderstand entgegensetzt, verhält sich die andere Zener-Diode in Bezug auf die Polarität des internen Spannungspulses entsprechend umgekehrt. Folglich ist gewährleistet, dass oberhalb des Betrages eines Durchbruchspannungswertes beide Zener-Dioden niederohmig leitend werden, unterhalb dieses Durchbruchspannungswertes jedoch eine Zener-Diode sperrend wirkt. Eine Spannungsbegrenzung kann oberhalb eines Durchbruchspannungswertes durch Ausbildung eines elektrischen Verbindungspfades zum Ausgleich der an weiteren elektronischen Bauteilen des medizinischen Geräts anliegenden Spannungen erreicht werden.

In einer weiteren Ausführungsform ist vorgesehen, dass der wenigstens eine hochspannungsfeste Kondensator eine Kapazität von weniger als 200 µF aufweist, insbesondere weniger als 100 µF und vorzugsweise von weniger als 50 µF. Die bezeichneten Kapazitäten sind gut für die weitgehend widerstandslose Ausgabe vorbestimmter Spannungspulse einer Nutzspannung in Stimulatoren und Herzschrittmachern geeignet.

Es ist vorgesehen, dass die Schnittstelle des medizinischen Gerätes eine bipolare Schnittstelle ist und jeder Pol dieser Schnittstelle mit einem überspannungsfesten Kondensator abgeschlossen ist. Bipolare Schnittstellen werden typischerweise bei Stimulatoren und Herzschrittmachern verwendet. Infolge des Abschlusses jedes Pols der Schnittstelle mit einem hinreichend spannungsfesten Kondensator wird bei Anliegen von externen Spannungssignalen mit gleicher zeitlicher Anstiegs- und/oder Abklingcharakteristik, wie etwa im Falle einer typischen Defibrillationsanwendung, jeweils ein interner Spannungspuls generiert, welcher für beide Pole ein weitgehend gleiches Zeitverhalten aufweist. Weiterhin ist gewährleistet, dass jedes in die bipolare Schnittstelle eingekoppeltes Überspannungssignal in einen internen Spannungspuls überführt wird.

Überdies kann in einer weiteren Ausführungsform vorgesehen sein, dass die Begrenzungsmittel wenigstens einen Schalter umfassen, welcher durch einen Schwellenwertdiskriminator bei Überschreiten eines vorbestimmten Spannungsbetrages des wenigstens einen internen Spannungspulses in eine Schließstellung überführt wird. Dabei kann ein elektrischer Verbindungspfad für einen Ausgleich der an weiteren elektronischen Bauteilen des medizinischen Geräts anliegenden Spannungen ausgebildet werden. Ein entsprechendes Überspannungsschutzelement kann somit durchaus mit einfachen elektronischen Schaltmitteln unter Verwendung von aus dem Stande der Technik bekannten Schwellenwertdiskriminatoren mehrteilig ausgeführt sein.

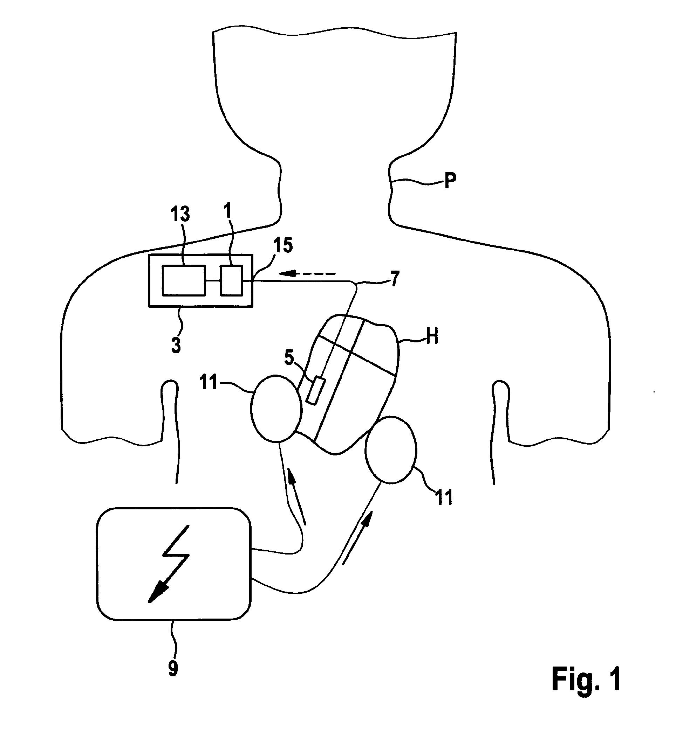

Fig. 1 zeigt das Vorsehen eines Überspannungsschutzelements 1 an einem implantierten Herzschrittmacher 3. Der Herzschrittmacher weist zur elektrischen Verbindung mit einer Körper-Gerät-Schnittstelle 5, welche hier als in das Herz H des Patienten P eingeführte Elektrode ausgeführt ist, eine Schnittstellenzuleitung (Elektrodenleitung) 7 auf. Die Elektrode (Körper-Gerät- Schnittstelle) 5 wird mit von dem Herzschrittmacher 3 erzeugten Spannungsimpulsen zur Herzmuskelreizung versorgt, welche an den Herzmuskel angelegt wird. Alternativ können mittels der Körper-Gerät-Schnittstelle 5 auch Herzsignale erfasst werden und über die Schnittstellenzuleitung 7 zum Herzschrittmacher geführt werden. Die Schnittstellenzuleitung 7 ist am Herzschrittmacher mit dem Überspannungsschutzelement 1 versehen.

Weiterhin zeigt Fig. 1 einen externen Defibrillator 9, der zwei weitere, als externe Defibrillationselektroden 11 ausgebildete Körper-Gerät-Schnittstellen umfasst. Kommt es zur Anwendung des Defibrillators am Patienten, wird über die Defibrillationselektroden 11 ein Spannungspuls auf die Brust des Patienten aufgebracht. Die Ausgabe von Schockimpulsen ist durch zwei Pfeile symbolisiert, welche vom Defibrillator 9 zu den beiden Defibrillationselektroden 11 führen. Aufgrund der örtlichen Nähe der Defibrillationselektroden 11 und der Elektrode 5 des Herzschrittmachers 3 zum Zeitpunkt der Abgabe des Spannungspulses sowie der Leitfähigkeit des Körpergewebes wird ein Teil der Defibrillationsenergie über die Körper-Gerät-Schnittstelle 5 des Herzschrittmachers in die Schnittstellenzuleitung 7 eingekoppelt und gelangt zum Überspannungsschutzelement 1, wie durch gestrichelt dargestellten Pfeil verdeutlicht wird. Aufgrund der Wirkung des Überspannungsschutzelements 1 wird jedoch nur ein geringer Anteil der Defibrillationsenergie in den Herzschrittmacher 3 eingekoppelt und dieser geschützt.

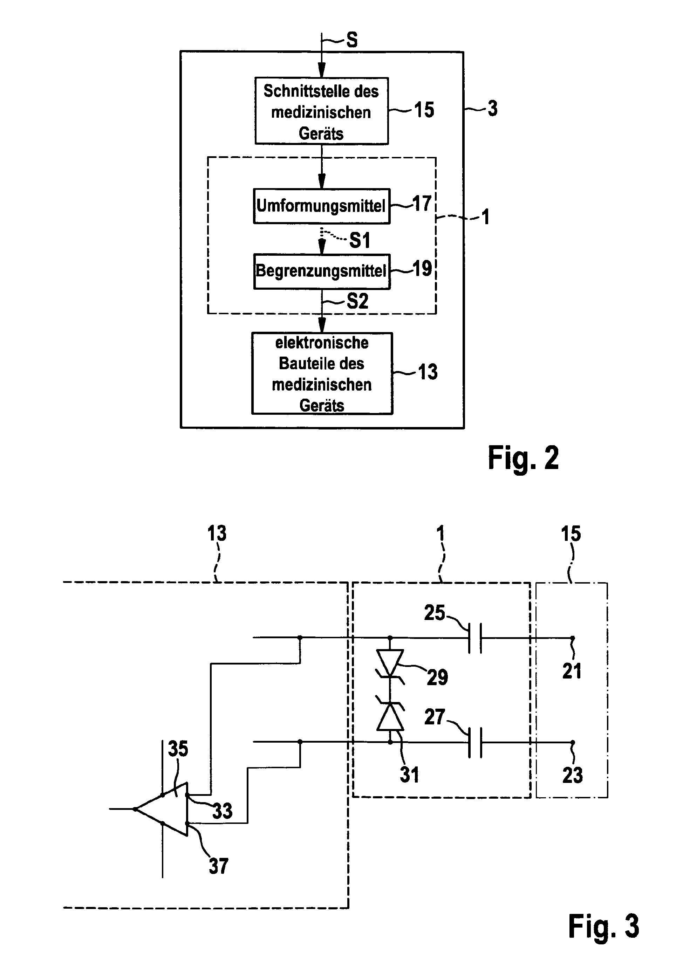

Fig. 2 zeigt ein Blockdiagramm zur Veranschaulichung der Funktion des Überspannungsschutzelements 1 zur Wandlung eines externen Überspannungssignals in einem medizinischen Gerät 3. Liegt ein externes Überspannungssignal S mit zeitlicher Anstiegs- und/oder Abklingcharakteristik an einer Schnittstelle 15 des Gerätes 3 wird zunächst das Signal in die Schnittstelle eingekoppelt. Umformungsmittel 17 des Überspannungsschutzelements 1 sind dazu ausgebildet, das externe Überspannungssignal S in einen internen Spannungspuls S1 zu überführen.

Entsprechend dem Betrag des internen Spannungspulses können Begrenzungsmittel 19 des Überspannungsschutzelements 1 die relative Amplitude des internen Spannungspulses, welche an weiteren elektronischen Bauteilen 13 des medizinischen Geräts 3 anliegen kann, auf einen vorbestimmten Grenzwert S2 begrenzen. Hierbei werden in einer Ausführungsform die Begrenzungsmittel 19 oberhalb eines vorbestimmten Spannungsschwellenwertes im Wesentlichen niederohmig stromleitend und erzeugen damit einen Strompfad zum Ausgleich der an den elektronischen Bauteilen des Geräts 13 anliegenden Spannungen und bewirken damit die Begrenzung der Amplitude.

Liegt die Amplitude des internen Spannungspulses unterhalb des durch die Begrenzungsmittel 19 vorbestimmten Grenzwerts, so verbleiben die Begrenzungsmittel 19 in einem weitgehend nicht leitenden Zustand. Die an den elektronischen Bauteilen 13 anliegende Spannung S2 entspricht dabei weitgehend dem Betrag der Amplitude des internen Spannungspulses S1, bewirkt dann jedoch keine Schädigung der elektronischen Bauteile 13, etwa durch einen Spannungsdurchschlag.

Fig. 3 zeigt ein Überspannungsschutzelements 1 an der Schnittstelle 15 eines medizinischen Geräts 3. Die Schnittstelle 15 ist eine bipolare Schnittstelle mit einem ersten Eingang 21 und einem zweiten Eingang 23. An der Schnittstelle befindet sich das Überspannungsschutzelement 1, welches den Eingängen 21 und 23 in Reihe nachgeschaltet einen ersten Kondensator 25 und einen zweiten Kondensator 27 als Umformungsmittel aufweist. Die Kondensatoren vermögen ein extern anliegendes Überspannungssignal mit zeitlicher Anstiegs- und/oder Abklingcharakteristik in einen eingekoppelten internen Spannungspuls zu überführen. Nachgeschaltet sind zwei Zener-Dioden 29 und 31, welche in entgegengesetzter Polung in Reihe geschaltet sind und beide Eingangsleitungen elektrisch verbinden. Der Kondensator 25 ist mit dem ersten Eingang 33 des Operationsverstärkers 35 verbunden, der Kondensator 27 mit dem zweiten Eingang des Operationsverstärkers 35. Der Operationsverstärker 35 ist hier nur stellvertretend für sonstige elektronische Bauteile zu verstehen, welche in der Schaltungsanordnung vorgesehen sein können.

Liegt nun eine externe Spannung an den Eingängen 21 und 23, mit positivem Potential am Eingang 21, wird durch den ersten Kondensator 25 ein positiver interner Spannungspuls generiert. Ist der Betrag des intern generierten positiven Spannungspulses größer als die Durchbruchspannung der Zener-Diode 29, wird diese niederohmig leitend. Die Zener-Diode 31 ist für diesen positiven Spannungsimpuls in Flussrichtung geschaltet, so dass ein Stromfluss über den Kondensator 27 an den zweiten Eingang 23 erfolgt. An den Anschlüssen 33 und 37 des Operationsverstärkers wird somit die Spannung auf die Summe der Durchbruch- und Flussspannung der Zener-Dioden begrenzt. Die Zener-Dioden 29 und 31 sind gemäß ihrer Durchbruchspannung vorteilhaft so auszuwählen, dass die maximal entstehende Spannungsdifferenz keine Beschädigung des Operationsverstärkers 35 verursacht.

Weiterhin ist dem Fachmann klar, dass bei Einkopplung eines internen Spannungspulses mit umgekehrter Polarität das Funktionsprinzip der vorliegenden Ausführungsform des Überspannungsschutzelementes 1 nicht verändert wird. Lediglich die beiden Zener-Dioden 29 und 31 vertauschen in Folge der Polaritätsänderung ihre Durchlass- und Sperreigenschaften. Es handelt sich bei der vorliegenden Anordnung der beiden Zener-Dioden 29 und 31 folglich in diesem Sinne um polaritätsunabhängige, symmetrische Begrenzungsmittel.

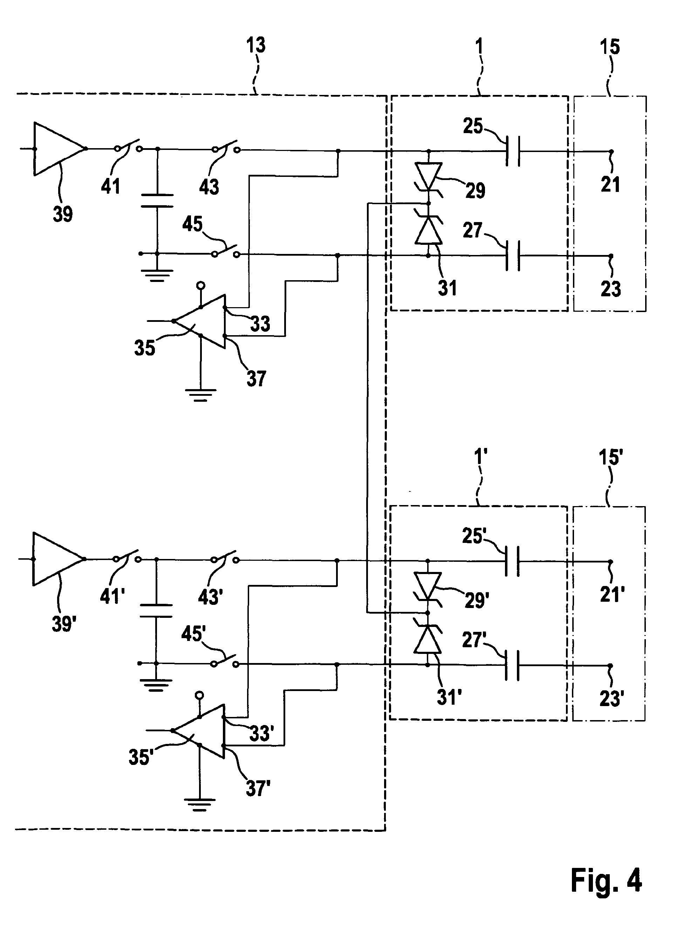

Fig. 4 zeigt ein erfindungsgemäßes Überspannungsschutzelement 1, welche auf der in Figur 3 dargestellten Schaltung beruht. Abweichend von jener Ausführungsform sind in der vorliegenden Ausführung jedoch zwei bipolare Schnittstellen 15 und 15' vorgesehen, welche beide jeweils durch ein Überspannungsschutzelement 1 bzw. 1' abgeschlossen sind, die im Grundaufbau dem in Figur 3 dargestellten Überspannungsschutzelement 1 entsprechen und daher hier nicht nochmals beschrieben werden.

Die beiden jeweils zwei Zener-Dioden 29, 31 bzw. 29', 31' umfassenden Begrenzungsmittel der beiden Überspannungsschutzelemente 1, 1' sind der in der Figur gezeigten Leitungsverschaltung derart verbunden, dass die die jeweils erste Zener-Diode 29, 29' und die jeweils zweite Zener-Diode 31, 31' verbindenden Leiterabschnitte auf ein gleiches Potential gelegt werden. Folglich werden in eine der beiden Überspannungsschutzelemente eingekoppelte externe Spannungssignale nicht nur über die einem Überspannungsschutzelement 1 bzw. 1' zugeordneten Leitungsabschnitte in ihren Potentialen ausgeglichen, sondern zudem über die beiden Überspannungsschutzelementen zugeordneten Leitungsabschnitte.

Neben den bereits beschriebenen Schaltungsbauteilen umfasst die in Fig. 4 dargestellte Schaltungsanordnung zwei weitere Verstärkerstufen 39 und 39', welche durch insgesamt sechs Schalter 41, 43 und 45 bzw. 41', 43' und 45' selektiv den einzelnen Eingängen 21 und 23 bzw. 21' und 23' zugeschaltet werden können. Da die Beträge der von den beiden Verstärkerstufen 39 und 39' ausgegebenen gepulsten Nutzsignale unterhalb der Durchbruchspannung der Zener-Dioden liegen, werden sie über die jeweiligen Schnittstellen, welche nun als Ausgänge fungieren, ausgegeben. Die beiden Operationsverstärker 35, 35' hingegen können zur Messwertverstärkung von elektrischen Messsignalen dienen, welche über die beiden Schnittstellen 15, 15' eingekoppelt werden.

Die Ausführung der Erfindung ist nicht auf die oben erläuterten Beispiele und hervorgehobenen Aspekte beschränkt, sondern im Rahmen des Schutzbereiches der anhängenden Ansprüche auch in einer Vielzahl abgewandelter Ausführungen möglich.

## Patentansprüche

1. Überspannungsschutzelement als Bestandteil
eines zum Einsatz an oder in einem menschlichen oder tierischen Körper vorgesehenen medizinischen Gerätes (3) zur Reduzierung einer Energieaufnahme bei Anliegen eines externen Überspannungssignals mit zeitlicher Anstiegs- und/oder Abklingcharakteristik an mindestens zwei Schnittstellen (15, 15') des medizinischen Gerätes (3),
umfassend Umformungsmittel (17), welche dazu ausgebildet sind, das externe Überspannungssignal an den Schnittstellen (15, 15') in einen internen Spannungspuls zu überführen, und Begrenzungsmittel (19), welche dazu vorgesehen sind, einen Spannungsabfall an wenigstens einem elektronischen Bauteilen des medizinischen Geräts auf einen vorbestimmten Grenzwert zu begrenzen,
wobei die Begrenzungsmittel (19) wenigstens zwei parallel zur jeweiligen Schnittstelle (15, 15') und zueinander in Reihe geschaltete Zener-Dioden (29, 31; 29', 31') umfassen, und von denen eine Zener-Diode in Sperrrichtung und eine in Durchlassrichtung geschaltet ist,
**dadurch gekennzeichnet, dass** die beiden jeweils wenigstens zwei Zener-Dioden (29, 31 bzw. 29', 31') umfassenden Begrenzungsmittel (19) derart verbunden sind, dass die die jeweils erste Zener-Diode (29, 29') und die jeweils zweite Zener-Diode (31, 31') verbindenden Leiterabschnitte auf ein gleiches Potential gelegt werden.

2. Überspannungsschutzelement nach Anspruch 1, **dadurch gekennzeichnet, dass** die Umformungsmittel (17) den Begrenzungsmitteln (19) in Bezug auf die Ausbreitungsrichtung des externen Überspannungssignals an der Schnittstelle (15) in Reihe vorgeschaltet sind.

3. Überspannungsschutzelement nach einem der vorhergehenden Ansprüche, **dadurch gekennzeichnet, dass** die Umformungsmittel (17) wenigstens einen hochspannungsfesten Kondensator umfassen.

4. Überspannungsschutzelement nach Anspruch 3, **dadurch gekennzeichnet, dass** der wenigstens eine hochspannungsfeste Kondensator eine Kapazität von weniger als 200 µF aufweist, insbesondere weniger als 100 µF und vorzugsweise von weniger als 50 µF.

5. Überspannungsschutzelement nach einem der vorhergehenden Ansprüche, **dadurch gekennzeichnet, dass** die Schnittstellen (15, 15') des medizinischen Gerätes (3) bipolare Schnittstellen sind und jeder Pol dieser Schnittstellen (15, 15') mit einem hochspannungsfesten Kondensator abgeschlossen ist.

## Claims

1. Overvoltage protection element as part of
a medical device (3) intended for use on or in a human or animal body for reducing power consumption when an external overvoltage signal with temporal rise and/or decay characteristic is present on at least two interfaces (15, 15') of the medical device (3),
comprising conversion means (17), which are designed to transform the external overvoltage signal at the interfaces (15, 15') into an internal voltage pulse, and limiting means (19), which are provided to limit a voltage drop on at least one electronic component of the medical device to a predefined limit value,
the limiting means (19) comprising at least two Zener diodes (29, 31; 29', 31') connected in parallel to the respective interface (15, 15') and connected in series to each other, of which one Zener diode is connected in the reverse direction and one in the forward direction,
**characterized in that** the two limiting means (19), which each comprise at least two Zener diodes (29, 31 or 29', 31), are connected such that the conductor sections connecting the first Zener diode (29, 29') and the second Zener diode (31, 31'), respectively, are connected to the same potential.

2. The overvoltage protection element according to claim 1, **characterized in that** the conversion means (17) are connected in series upstream of the limiting means (19) with respect to the propagation direction of the external overvoltage signal at the interface (15).

3. An overvoltage protection element according to any one of the preceding claims, **characterized in that** the conversion means (17) comprises at least one high voltage resistant capacitor.

4. The overvoltage protection element according to claim 3, **characterized in that** the at least one high voltage resistant capacitor has a capacitance of less than 200 µF, in particular less than 100 µF, and preferably less than 50 µF.

5. An overvoltage protection element according to any one of the preceding claims, **characterized in that** the interfaces (15, 15') of the medical device (3) are bipolar interfaces and each pole of these interfaces (15, 15') is terminated by a high voltage resistant capacitor.

## Revendications

1. Élément de protection contre les surtensions, en tant que composant d'un appareil médical (3) destiné à être utilisé sur ou dans un corps humain ou animal, pour réduire la réception d'énergie lors de l'application d'un signal de surtension externe avec une caractéristique temporelle d'augmentation et/ou de désactivation sur au moins deux interfaces (15, 15') de l'appareil médical (3), comprenant des moyens de conversion (17) conçus pour convertir le signal de surtension externe en une impulsion de tension interne sur les interfaces (15, 15'), ainsi que des moyens de limitation (19) prévus pour limiter une chute de tension à une valeur seuil prédéterminée sur au moins une pièce électronique de l'appareil médical,
dans lequel les moyens de limitation (19) comprennent au moins deux diodes Zener (29, 31 ; 29', 31') branchées en parallèle par rapport à chacune des interfaces (15, 15') et en série l'une par rapport à l'autre, parmi lesquelles une diode Zener est branchée dans le sens de verrouillage et l'autre dans le sens de passage,
**caractérisé en ce que** les deux moyens de limitation (19) comprenant chacune au moins deux diodes Zener (29, 31 ; 29', 31') sont reliés de telle manière, que les sections conductrices reliant respectivement la première diode Zener (29, 29') et la deuxième diode Zener (31, 31') sont au même potentiel.

2. Élément de protection contre les surtensions selon la revendication 1, **caractérisé en ce que** les moyens de conversion (17) sont branchés en série en amont des moyens de limitation (19) par rapport au sens de propagation du signal de surtension externe sur l'interface (15).

3. Élément de protection contre les surtensions selon l'une des revendications précédentes, **caractérisé en ce que** les moyens de conversion (17) comprennent au moins un condensateur résistant aux hautes tensions.

4. Élément de protection contre les surtensions selon la revendication 3, **caractérisé en ce que** l'au moins un condensateur résistant aux hautes tensions a une capacité inférieure à 200 µF, en particulier inférieure à 100 µF et de préférence inférieure à 50 µF.

5. Élément de protection contre les surtensions selon l'une des revendications précédentes, **caractérisé en ce que** les interfaces (15, 15') de l'appareil médical (3) sont des interfaces bipolaires, et chaque pôle de ces interfaces (15, 15') est isolé par un condensateur résistant aux hautes tensions.
